# NEW EUROPEAN PATENT SPECIFICATION

(11) **EP 0 348 015 B2**
(45) Date of publication and mention of the opposition decision: **06.08.1997**
(45) Mention of the grant of the patent: 01.09.1993
(21) Application number: 89201951.4
(22) Date of filing: 16.06.1988
(51) Int. Cl.: A61K 7/07

(54) **Hair preparation**
Haarkosmetisches Präparat
Composition pour les cheveux

(30) Priority: 18.06.1987 JP 152376/87; 18.06.1987 JP 152377/87
(43) Date of publication of application: 27.12.1989
(62) Divisional of application: 88305486.8
(73) Proprietor: Kao Corporation, Chuo-Ku Tokyo 103 (JP)
(72) Inventor: Yoshihara, Toru, Funabashi-shi Chiba (JP); Kawase, Jiro, Funabashi-shi Chiba (JP); Fukuyama, Yukihiro, Wakayama (JP)
(74) Representative: Bannerman, David Gardner

(56) References cited:
- EP-A- 060 611
- BE-A- 671 120
- DE-A- 2 301 660
- DE-B- 1 040 750
- FR-A- 2 549 369
- GB-A- 1 359 492
- JP-A-57 080 499
- US-A- 4 237 112
- US-A- 4 489 048
- US-A- 4 664 910
- ZA-A- 670 815
- Cosmetic and toileteries formulations, Sect. X, Shampoos, pp. 408-414, E.W Flick, Noyes Publications, 1984
- A formulary of cosmetic preparations, pp. 87-91, M. Ash et al, Chem. Publ. Co., 1977
- The thesaurus of Chem. Prod., 2nd ed., M. Ash et al, 1992
- Cosmetic additives - an industrial guide, p. 786, E.W. Flick, Noyes Publ., 1991
- Résumé Chem. Abs., CA 81(20), 126683r
- Résumé Chem. Abs., CA 81(18), 111464w
- Résumé Chem. Abs. CA 70(14), 59152z
- Résumé Chem. Abs. CA 66(14), 58807x
- Résumé Derwent 82-53280E(26)
- Résumé Chem. Abs. CA 98(6), 40404q
- Pharm. Weekbl. 117(48), p. 1122-3, H.H.J. Allart, 1982

## Description

The present invention relates to hair preparations in solid form or in the form of a lotion having a high sebum absorptivity and providing comfort in use and of reducing greasiness of the scalp and hair.

More specifically, it relates to hair preparations comprising an anti-dandruff agent and a specified oil-absorptive substance being capable of absorbing sebum.

Although sebum makes the skin and hair glossy and smooth when the correct amount is selected it makes the skin and hair greasy and sticky when secreted excessively. Further, a makeup applied to the face is impaired by the sebum. In addition, the apparent volume of the hair is reduced by the sebum to make the retention of' an attractive hairstyle impossible. Although the excess sebum can be easily removed by washing, the amount becomes excessive again within a few hours on the skin and within a few days on the scalp and hair and, therefore, repeated washing is necessitated.

Under these circumstances, studies are in progress for the purpose of physiologically controlling the secretion of the sebum or physically absorbing the excessive sebum with a powder.

However, the former method is not employed practically, since no safe and effective preparation has been found yet.

Although the latter method wherein the sebum is physically absorbed by a powder has no serious problem of safety, the powder so far investigated displays insufficient absorptivity and do not provide a feeling of comfort during use.

For example, a process for controlling the secretion of sebum with an inorganic powder such as a clay mineral or an oxide is described in Japanese Patent Laid-Open No.65807/1986. However, such inorganic substances usually have a defect that their oil absorptivity is seriously reduced by sweat or dirt.

A method of reducing seborrhoea with silylated silica gel is described in Japanese Patent Laid-Open No.144710/1984. In this method, the surface of the inorganic powder particles are made hydrophobic and the sebum is absorbed in the interparticle spaces in aggregates of the primary particles. A high oil level of absorptivity cannot be expected by this method.

U.S. Patent No. 4,489,058 discloses a method of absorbing sebum by a copolymer of styrene with stearyl methacrylate having high compatibility with sebum by swelling in order to keep the skin free from acne. Although this method is excellent in that sebum can be absorbed even in the presence of sweat or dirt, the level of absorption is still insufficient and the feel after absorption of the sebum is poor, since the sebum is absorbed by mere swelling. Another defect of this method is that the copolymer scarcely adheres to hair, since it has an average particle diameter of around 150 µm.

Various anti-dandruff agents are incorporated in hair preparations such as shampoos, rinses and hair tonics in order to prevent dandruff formation or itch. All of them have an antibiotic activity and act by dissolving the corneous layer or controlling the metabolic rate of this layer. As a result, dandruff formation on the scalp is prevented. However, it has been pointed out that daily use of an anti-dandruff agent exerts a significant effect on the physical properties of the hair. Namely, the hair becomes greasy and sticky soon after shampooing [see, for example, International Journal of Cosmetics Science, 5, 77 (1983)].

The mechanism of the phenomenon whereby hair becomes greasy upon daily use of the anti-dandruff agent-containing composition has not been fully elucidated yet. In this connection, no anti-dandruff composition also having an effect of inhibiting or retarding the formation of greasiness has been developed.

As for special oil-absorptive substances capable of absorbing sebum, for example, colloidal silica is described in Japanese Patent Publication No.20793/1971 and a combination of a hydroxycholanic acid derivative and a powdery oil absorbent is disclosed in European Patent No. 58000. They are used as raw materials for skin cosmetics. However, no anti-dandruff composition usable for controlling or retarding the realization of greasiness has been reported.

Dry shampoo are also known. For example, French Patent Application No. 2549369 describes a dry shampoo based on polyvinylpyrrolidone powder, which is applied to the hair in the form of an aerosol and which is then brushed out.

After clinical analysis of the tendency of sebum to diffuse towards the hair as a result of the repeated use of ordinary anti-dandruff agents made for the purpose of overcoming the problem of the hair becoming greasy at an early stage when an ordinary anti-dandruff agent is used every day, the inventors have found that the secreted sebum is distributed to the surface of the scalp and then to the hair after a few days, more precisely the sebum reaches saturation on the surface of the scalp within one or two days and then begins to diffuse towards hair, that the fragments of corneous layer on the surface of the scalp, (i.e. dandruff), play an important role as a sebum reservoir structure, that a decrease in the dandruff due to the effect of an anti-dandruff agent brings about a decrease in the amount of the sebum capable of being stored on the scalp surface and thus an increase in the diffusion rate of the sebum towards the hair, and that this phenomenon is observed from one day after shampooing.

After intensive investigations made for the purpose of overcoming the above-described defects, we have found that the problems can be solved by using a porous, oil-absorptive vinyl polymer. The present invention has been completed on the basis of this finding.

The inventors have supposed that if a new sebum reservoir structure were formed on the scalp and/or hair surface as a substitute for the fragments of corneous layer (i.e. dandruff), which are aesthetically undesirable, the development of greasiness can be delayed or eliminated. After intensive investigations made for the purpose of developing a means of forming such a sebum reservoir structure, we have found that when an anti-dandruff composition comprising a carrier far smaller than the correct layer fragments and having a sebum absorptivity higher than that of said fragments is used, dandruff and itch can be prevented, controlled and/or relieved and, in addition, the development of greasiness can be retarded.

The present invention has been completed on the basis of the above-described findings.

According to the invention we therefore provide . a hair preparation in solid form or in the form of a lotion which comprises 0.01 to 10 percent by weight of an anti-dandruff agent, 0.05 to 5 percent by weight of a cross-linked oil absorptive porous vinyl polymer in the form of particles having an average particle size of 0.005 to 30 µm and having a squalene absorption of at least 1 ml/g in the dried state and the balance of a carrier.

The hair preparation of the invention preferably comprises an oil-absorptive polymer of a vinyl monomer having a solubility parameter of 7 to 10, and a carrier.

It is preferred that the oil-absorptive polymer has such a size distribution that it contains 90 percent by weight or more of those having particle sizes in the range of 0.005 to 30 µm.

At first, the composition comprising the oil-absorptive polymer will be explained below in detail.

The present invention provides hair preparations characterised by comprising an oil-absorptive polymer having a squalene absorption of at least 1 mℓ/g which is prepared by polymerizing one or more vinyl monomers having a solubility parameter of 7 to 10 and making the resulting polymer porous.

The oil-absorptive polymer for use in the present invention preferably has an average particle diameter of 0.005 to 30 µm. Preferably the polymer comprises at least 90 wt.% of particles having a diameter of 0.005 to 30 µm and is crosslinked. The porous polymer is preferably prepared by dissolving the monomer in a nonpolymerizable organic solvent, suspending, dispersing or emulsifying the solution in water, conducting the polymerization and then removing the organic solvent.

The polymerization can be conducted by any known method such as suspension, dispersion or emulsion polymerization. Suspension polymerization is recommended when a polymer having a particle diameter of 10 µm or larger is desired. Dispersion polymerization or emulsion polymerization are recommended when a polymer having a particle diameter of 1 to 10 µm or 1 µm or less, respectively, is desired. From the viewpoints of the feel of the skin and hair after application of the preparation and adhesion of the particles, smaller particles are preferred. However, emulsion polymerization has the disadvantage that the surfactant which is used in a relatively large amount cannot be removed easily.

The polymer can be made porous by removing nonpolymerizable substances such as an organic solvent, plasticizer and linear polymer contained in the polymerization solution after the completion of the polymerization. A particularly preferred process comprises dissolving the monomer in a nonpolymerizable organic solvent which is a good solvent for the monomer but a poor solvent for the resulting polymer, suspending, dispersing or emulsifying the monomer solution in water, polymerizing it and removing the organic solvent after the completion of the polymerization.

The monomers usable in the present invention are preferably vinyl monomers having a solubility parameter of 7 to 10. They include, for example, esters of methacrylic acid with higher alcohols having 8 to 24 carbon atoms, esters of acrylic acid with higher alcohols having 8 to 24 carbon atoms, styrene, styrene derivatives having a straight-chain or branched hydrocarbon substituent having 1 to 12 carbon atoms, vinyl esters of unsaturated carboxylic acids having 8 to 20 carbon atoms, acrylic acid, methacrylic acid and diolefins having 4 to 6 carbon atoms. If the solubility parameter of the vinyl monomer is outside the range of 7 to 10, the compatibility of the product with sebum is inferior.

These monomers can be used either alone or in the form of a combination of two or more of them.

When a monomer having a long-chain alkyl group, such as stearyl methacrylate, is used alone, the polymer will have a low glass transition point (Tg) and a low porosity, though it has a high compatibility with sebum. Thus, the monomer is preferably copolymerized with a comonomer capable of forming a polymer having a Tg of 100°C (373°K) or higher, such as styrene.

Examples of particularly preferred monomers include esters of higher alcohols having 8 to 24 carbon atoms, such as stearyl alcohol, with acrylic or methacrylic acid. Although they can be homopolymerized, it is preferred to copolymerize the monomer with styrene or styrene substituted with a straight-chain or branched alkyl group having 1 to 12 carbon atoms and capable of forming a polymer having a Tg of 100°C (373°K) or higher in order to further increase the porosity and oil absorptivity (refer to Preparation Examples 1 and 2). The weight ratio of the acrylate or methacrylate to styrene or its derivative is preferably at least 30/70. When this ratio is less than 30/70, the polymer becomes hard and, therefore, the feel thereof becomes impaired.

To obtain an excellent touch, it is preferred that the oil-absorptive polymer according to the present invention is swellable with sebum but insoluble therein. Therefore, the polymer is preferably crosslinked. Crosslinking is conducted by a process wherein a polyfunctional monomer is added during the polymerization, a crosslinking process or a self-crosslinking process. The polyfunctional monomers include, for example, polyvinyl `aromatic compounds such as divinylbenzene, polyvinyl heterocyclic compounds such as divinylpyridine, dimethacrylates such as ethylene glycol dimethacrylate and triethylene glycol dimethacrylate, and diacrylates such as ethylene glycol diacrylate and triethylene glycol diacrylate. The amount of the crosslinking agent is preferably 50 wt.% or below based on the polymer. When it exceeds 50 wt.%, the swellability of the polymer is seriously inhibited. A particularly preferred amount of the crosslinking agent is 0.01 to 50 wt.% based on the total polymer.

The organic solvents usable for making the polymer porous in the present invention include, for example, aromatic compounds such as toluene and benzene, esters such as ethyl acetate and butyl acetate, alcohols such as isoamyl alcohol and methylisobutyl carbinol, saturated hydrocarbons such as n-hexane, n-octane and n-dodecane, and halogenated solvents such as dichloroethane and trichloroethylene. Among them, those which are good solvents for the vinyl monomers having a solubility parameter of 7 to 10 and which are poor solvents for the polymers prepared from the monomers in the present invention are particularly preferred. The preferred solvents include, for example, aliphatic hydrocarbons such as hexane, octane and dodecane, and aromatic hydrocarbons such as toluene and benzene. The weight ratio of the monomer to the organic solvent is preferably 1/1 to 1/4.

The plasticizers are, for example, dioctyl phthalate and dibutyl adipate. The linear polymers are, for example, polystyrene and polyvinyl acetate.

The polymerization initiators are ordinarily used oil-soluble ones. They include, for example, peroxides and azo compounds such as benzoyl peroxide, lauroyl peroxide, 2,2'-azobisisobutyronitrile, 2,2' azobis-(2,4-dimethylvaleronitrile), o-chlorobenzoyl peroxide and o-methoxybenzoyl peroxide.

The dispersion and emulsion stabilizers usable in the present invention are those usually used. They include, for example, water-soluble polymers such as starch, hydroxyethylcellulose, carboxymethyl-cellulose, polyvinylpyrrolidone, polyvinyl alkyl ethers and polyvinyl alcohols; difficultly water-soluble inorganic salts `such as barium sulfate, calcium sulfate, barium carbonate, calcium carbonate, magnesium carbonate and calcium phosphate; and surfactants such as sodium lauryl sulfate, sodium cetyl sulfate and sodium polyoxyethylene lauryl ether sulfate.

The monomer solution comprising the monomer(s), diluent (an agent for making the polymer porous) and polymerization initiator is dispersed or suspended in water in the presence of the above-mentioned dispersion or emulsion stabilizer by a known process to form droplets and then polymerization is conducted to prepare the intended porous polymer. In this process, the monomer solution is dispersed or suspended by an ordinary method such as an ordinary stirring method with any of various ordinary stirrers or a forced stirring method with a homogenizer or by irradiation with ultrasonic waves. These means are selected suitably to obtain particles having intended particle diameters. The proper polymerization temperature varies depending on the polymerization initiator used. It is `usually in the range of 20 to 95°C. As a matter of course, the polymerization temperature must be lower than the boiling points of the monomer(s) and diluent under atmospheric pressure.

After the completion of the polymerization, the polymer particles are separated by filtration. The aqueous phase is removed. The polymer particles are washed with water and/or with a solvent in order to replace the diluent having a high boiling point with a solvent having a lower boiling point. The product is then converted into powder by an ordinary method such as spray-drying or reduced-pressure drying.

Before the polymerization, the mixture is in the form of droplets of a homogeneous solution comprising the monomer(s), diluent and initiator. As the polymerization proceeds, however, the diluent which is a poor solvent for the polymer(s) causes microscopic phase separation and is then vaporized (desorbed) during drying under reduced pressure to form the intended porous polymer.

The oil absorption of the oil-absorptive polymer is determined by the "Method of Measurement of Oil Absorption of Pigment" stipulated in JIS K 5101 (1978) using squalene as the oil. In this method, 1 g of the powder is placed on a glass plate and kneaded by means of a spatula while squalene is gradually added dropwise thereto until the powder is wholly converted into a paste. The amount of squalene (mℓ) required per gram of the powder is referred to as the absorption. Although boiled linseed oil is used in JIS, squalene is used in the process of the present invention, since it is close to sebum. The absorption of the polymer according to the present invention is at least 1 mℓ/g, preferably at least 2 mℓ/g. The porosity can be determined in terms of the pore volume determined from the pore distribution measured by mercury porosimetry. The detail of this method is described in, for example, "Funtai Kogaku Binran" edited by Funtai Kogaku-kai and published by Nikkan Kogyo Shinbun-sha in 1986. The pore volume is at least 0.1 mℓ/g, preferably at least 0.15 mℓ/g.

The porous oil-absorptive polymer thus prepared can be dispersed in water, a lower alcohol or a mixture of them to form a dispersion usable as a hair lotion. The most preferred base of the hair preparation of the present invention is a mixture of water with ethanol in a weight ratio of 99/1 to 20/80, preferably 95/5 to 40/60. The amount of the oil-absorptive polymer in the hair lotion is preferably 0.05 to 5 wt.%, particularly 0.1 to 2 wt.%.

The lotion can contain further various water-soluble polymers in order to stabilize the dispersion or to dress the hair. They include monionic water-soluble polymers such as polyvinyl alcohol, polyvinylpyrrolidone and hydroxyethylcellulose; and anionic water-soluble polymers such as carboxymethyl-cellulose, crosslinked polyacrylic acid (carboxyvinyl polymer), xanthan gum and guar gum. These polymers are used in an amount of preferably 0.01 to 5 wt.%, particularly 0.05 to 1 wt.%.

The hair preparation of the present invention may contain, in addition to the above-described components, such other ordinary components in such amounts that they do not damage the effect of the present invention. They include, for example, polyhydric alcohols, e.g. glycerol, dipropylene glycol and 1,3-butylene glycol, nonionic surfactants, cationic surfactants, anionic surfactants, dyes, antiseptics, flavors, antioxidants, chelating agents, germicides, medicinal preparations, e.g. vitamins and hormones, astringents and U.V. absorbers.

The porous vinyl polymer contained in the hair preparation of the present invention has a far higher capacity for absorbing squalene than that of ordinary inorganic powders and water-insoluble polymers. Therefore, the hair preparation containing the porous vinyl polymer exhibits an excellent effect in reducing greasiness as will be shown in the following Examples.

Then the composition comprising an anti-dandruff agent and an oil-absorptive substance will be below explained in detail.

The present invention provides hair preparations characterized by comprising 0.01 to 10 wt.% of an anti-dandruff agent and 0.05 to 5 wt.% of an oil-absorptive substance in the form of fine particles and having a squalene absorption of at least 0.5 mℓ/g in a dried state.

The anti-dandruff agents usable in the present invention are well known ones. They include, for example, polyvalent metal salts of 2-mercaptopyridine N-oxide, colloidal sulfur, sulfur-containing amino acids and salts of them described in Japanese Patent Laid-Open No. 183614/1983, 2,2'-dithiobispyridine 1,1'-dioxide of the formula (I): 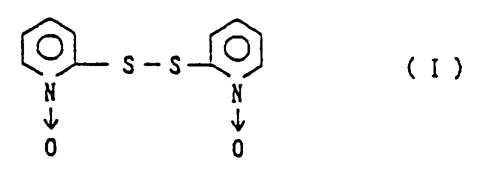 and hydrates of metal salts thereof such as magnesium sulfate thereof, 1-hydroxy-2-pyridone derivatives of the general formula (II): 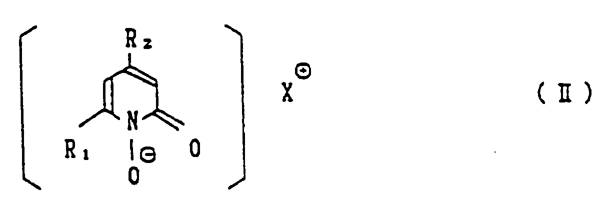 wherein R₁ represents an alkyl group having 1 to 17 carbon atoms, an alkenyl group having 2 to 17 carbon atoms, a cycloalkyl group having 5 to 8 carbon atoms, a bicycloalkyl group having 7 to 9 carbon atoms, a cycloalkylalkyl group in which the alkyl group has 1 to 4 carbon atoms and the cycloalkyl group may be substituted with an alkyl group having 1 to 4 carbon atoms, an aryl group, an aralkyl group in which the alkyl group has 1 to 4 carbon atoms, an arylalkenyl group in which the alkenyl group has 2 to 4 carbon atoms, an aryloxyalkyl group or an arylmercaptoalkyl group in which the alkyl group has 1 to 4 carbon atoms, a benzhydryl group, a phenylsulfonylalkyl group in which the alkyl group has 1 to 4 carbon atoms, a furyl or furylalkenyl group in which the alkenyl group has 2 to 4 carbon atoms (the aryl residue in each of the above-mentioned groups may be substituted with an alkyl or alkoxy group having 1 to 4 carbon atoms, a nitro group, a cyano group or a halogen atom), R₂ represents a hydrogen atom, an alkyl group having 1 to 4 carbon atoms, an alkenyl or alkynyl group having 2 to 4 carbon atoms, a halogen atom, a phenyl group or a benzyl group, and X represents a residue of an organic amine,
described in Japanese Patent Publication No. 39805/1983, salicylic acid and its derivatives, triethyl citrate described in Japanese Patent Laid-Open No. 180417/1983, indole derivatives of the general formula (III): 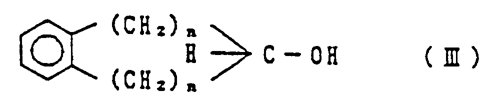 described in West German Patent No. 3142296, compounds of the general formula (IV): 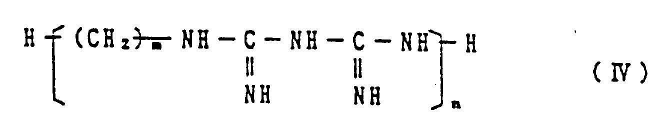 wherein m represents a number of 1 to 10 and
n represents a number of 1 to 6,
and salts of them described in West German Patent No. 012767, 2-oxotetrahydro-1,3,5-thiadiazine derivatives of the general formula (V): 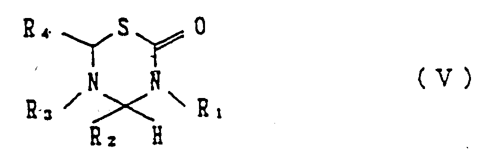 described in west German Patent No. 3022799, ω-(aminothiocarbonylmercapto)alkanoic acids described in Belgian Patent No. 2085728, compounds of the general formula (VI):

R-NHC(S)-S-(CH₂)ₙCOOH (VI)

and salts of them, quinone derivatives, selenium disulfide, phenol derivatives and coal tar.

Among them, the polyvalent metal salts of 2-mercaptopyridine, 2,2'-dithiobispyridine 1,1'-dioxideof the formula (I) and 1-hydroxy-2-pyridone derivatives of the general formula (II) are preferred from the viewpoints of the safety and effectiveness. Particularly preferred are zinc salt of 2-mercaptopyridine (zinc pyrithione), triethanolamine salt of 1-hydroxy-4-methyl-6-(2,4,4-trimethylpentyl)-2(1H)-oyridone (Piroctone Auramine) and 2,2'-dithiobispyridine 1,1'-dioxide. The zinc salt of 2-mercaptopyridine is used preferably in the form of fine particles at least 50 wt.% of which have a particle diameter of 0.2 µm or less and which have an average particle diameter of 0.2 µm or less as described in Japanese Patent Laid-Open Nos. 16972/1985, 16973/1985 and 224676/1985.

The oil-absorptive substance in the form of fine particles used in the present invention has a particle size (0.005 to 30 µm on average) far smaller than the ordinary fragments of corneous layer and an oil absorption comparable to that of such fragments in a dried -state (1 mℓ·squalene/g).

From the viewpoints of oil absorptivity, touch and adhesion to the skin, the porous vinyl polymer prepared from one or more vinyl monomers preferably has a solubility parameter of 7 to 10. Typical examples of the vinyl monomers having a solubility parameter of 7 to 10 include esters of methacrylic or acrylic acid with a higher alcohol having 8 to 24 carbon atoms, styrene and styrene derivatives having an alkyl substituent having 1 to 12 carbon atoms. A particularly preferred vinyl monomer is a mixture of the acrylate or methacrylate with styrene or styrene derivative in a weight ratio of 30/70 or higher. The porous polymer can be prepared by mixing the monomer or a mixture of the monomers with a nonpolymerizable organic solvent such as a volatile aliphatic hydrocarbon, e.g. n-hexane, n-octane or n-dodecane, or an aromatic hydrocarbon such as benzene or toluene in a weight ratio of the monomer(s) to the organic solvent of 1/1 to 1/4, subjecting the mixture to dispersion, suspension or emulsion polymerization in water and removing the nonpolymerizable organic solvent.

The polymer is crosslinked, preferably with a polyfunctional monomer, for example, a polyvinyl aromatic compound such as divinylbenzene, a polyvinyl heterocyclic compound such as divinylpyridine, a dimethacrylate such as ethylene glycol dimethacrylate or triethylene glycol dimethacrylate, or a diacrylate such as ethylene glycol acrylate or triethylene glycol acrylate. The amount of the crosslinking agent is preferably 50 wt.% or less based on the polymer. When it exceeds 50 wt.%, the swellability of the polymer is seriously reduced. A particularly preferred amount of the crosslinking agent is 0.01 to 50 wt.% based on the polymer.

The most preferred vinyl polymer used in the present invention is prepared by polymerizing a mixture of stearyl methacrylate, styrene, divinylbenzene and toluene in water and then making the formed polymer porous.

The oil absorption of the oil-absorptive fine particles is determined by the "Method of Measurement of Oil Absorption of Pigment" according to JIS K 5101 (1978) using squalene as the oil in the present invention. In this method, 1 g of the powder is placed on a glass plate and kneaded by means of a spatula while squalene is gradually added dropwise thereto until the powder is wholly converted into a paste. The amount of squalene (mℓ) required per gram of the powder is referred to as the absorption. Although boiled linseed oil is used in JIS, squalene is used in the process of the present invention, since it is close to sebum.

The oil absorption of typical, commercially available oil-absorptive substances determined by the above-mentioned method is shown in Table 1, wherein squalene is used as the oil.

**Table 1**

| Oil-absorptive substance | Oil absorption (mℓ/g) |
|---|---|
| synthetic phyllosilicate [Mg₃(Si₄O₁₁)H₂O·11H₂O] | 1.07 |
| ditto [K₂O·3Al₂O₃·6SiO₂] | 0.99 |
| synthetic platy silicate [Ca₄(Si₆O₁₆)(OH)·4H₂O] | 4.96 |
| ditto [Ca₄(Si₆O₁₆)(OH)(OH)] | 5.09 |
| dry synthetic platy silicate [Ca(Si₆O₁₆)(OH)] | 1.40 |
| porous colloidal silica having a pore diameter of 120 Å [SiO₂·nH₂O] | 1.52 |
| porous colloidal silica having a pore diameter of 60 Å [SiO₂·nH₂O] | 1.38 |
| Talc JA46A | 0.54 |
| Kaolin A | 0.72 |
| bentonite mainly comprising montmorillonite | 0.86 |
| spherical colloidal silica [Aerosil 200] | 5.00 |
| spherical colloidal silica [Aerosil 130] | 5.38 |
| porous nylon beads [Orgasol 2000D] | 0.80 |
| non-porous nylon beads [Toray Nylon SP500] | 0.57 |

The hair preparation of the present invention may be in either solid or liquid form. Usually a liquid suspension of the oil-absorptive substance in the form of fine particles is preferably used. The liquid in which the anti-dandruff agent and the oil-absorptive fine particles of the present invention are suspended is, for example, water, a lower alcohol or a mixture of them. When the hair preparation in solid form is applied to the scalp and hair and rubbed thereinto, it becomes liquid to uniformly and effectively spread the anti-dandruff agent and the oil-absorptive agent on the surfaces thereof. The solid hair preparation is desirably in the form of gel, paste or the like. It can be used easily when it contains a substantial amount of a solvent.

The alcohols used as the solvent are preferably lower monohydric alcohols and part of them may be replaced with a dihydric or polyhydric alcohol. The monohydric alcohols usable in the present invention are preferably ethanol and isopropanol. Mixtures of two or more components selected from the group consisting of ethanol, isopropanol, water and other monohydric, dihydric and polyhydric alcohols can also be used. The anti-dandruff agent can be suspended or dissolved also therein.

To keep the suspension of the oil-absorptive substance homogeneous, an organic gum can be used. The organic gums include, for example, carboxymethylcellulose, hydroxyalkylcellulose, carboxypolymethylene polyvinylpyrrolidone, acrylates, gelatin, dextrin, tragacanth, acacia, alginic acid, pectin and carrageenan.

The hair preparation of the present invention may contain various other components for various purposes. They include, for example, ordinary colorants such as dyes and pigments, flavors, vitamins hair growth promoting agents and surfactants.

The hair preparation of the present invention is a new one prepared on the basis of a new conceptior unlike ordinary anti-dandruff compositions. The hair preparation is effective in preventing dandruff and itch and also in preventing hair from becoming greasy, which were problems with the ordinary anti-dandruff compositions.

### Examples

The following Preparation Examples and Examples will further illustrate the present invention:

### Preparation Example 1

40 g of stearyl methacrylate, 40 g of divinylbenzene, 80 g of toluene, 2 g of benzoyl peroxide and 700 g of a 0.6% aqueous polyvinyl alcohol solution were placed in a 2-ℓ separable flask and mixed by means of a homomixer at 11,000 rpm for 5 min. Then the dispersion thus obtained was stirred at 300 rpm at 80°C for 8 h in a nitrogen atmosphere to conduct polymerization. After the completion of the polymerization, the reaction mixture was filtered. The filtration residue was washed with water and then with acetone and dried to obtain 40 g of a porous vinyl polymer having an average particle diameter of 6.7 µm.

According to the results of the determination of the pore distribution by mercury porosimetry, the polymer had pores of smaller than 347 Å on their particle surfaces and a pore volume of 0.15 mℓ/g.

### Preparation Example 2

20 g of stearyl methacrylate, 20 g of styrene, 40 g of divinylbenzene, 30 g of toluene, 90 g of n-dodecane, 0.8 g of benzoyl peroxide and 470 g of a 3.3% aqueous sodium dodecylsulfate solution were placed in a 2-ℓ separable flask and stirred at 300 rpm at 65°C for 9 h in a nitrogen atmosphere to conduct polymerization. After the completion of the poymerization followed by the same procedure as that of Preparation Example 1, 50 g of a porous vinyl polymer having an average particle diameter of 2.0 µm was obtained.

According to the results of the determination of the pore distribution by mercury porosimetry, the polymer had pores of smaller than 725 Å on their particle surfaces and a pore volume of 0.299 mℓ/g.

### Preparation Example 3

17 g of stearyl methacrylate, 17 g of divinylbenzene, 34 g of toluene, 1 g of potassium persulfate and 530 g of 1.0% aqueous polyoxyethylene (35 mol of ethylene oxide added) nonylphenyl ether were placed in a 2-ℓ separable flask and stirred at 300 rpm at 75°C for 8 h in a nitrogen atmosphere to conduct polymerization. After the completion of the polymerization followed by topping conducted under 100 to 150 mmHg for 2 to 3 h, a porous vinyl polymer was obtained in the form of an aqueous suspension. The average particle diameter of the polymer was smaller than 0.7 µm.

### Preparation Example 4

A porous vinyl polymer having an average particle diameter of 2.5 µm was prepared in the same manner as that of Preparation Example 2 except that 20 g of styrene was replaced with 20 g of t-butylstyrene and the amount of divinylbenzene was altered from 40 g to 0.4 g.

### Preparation Examples 5 and 6

A vinyl polymer was prepared in the same manner as that of Preparation Example 1 except that the amount of toluene was altered to 160 g (Preparation Example 5) or no toluene was used (Preparation Example 6).

The polymer thus prepared in Preparation Example 6 was a comparative one.

The oil absorptivity of the polymers prepared in the above Preparation Examples 1 to 6 was determined according to the above-mentioned method based on JIS K 5101. For comparison, the oil absorptivity of talc, kaolin and Polymer No. 14 in Example 1 of U.S. Patent No. 4489058 which has the highest oil absorptivity among all the polymers mentioned in the specification of this patent and which comprises 65 wt.% of t-butylstyrene, 35 wt.%. of stearyl methacrylate and 0.0125 wt.% of divinylbenzene as the crosslinking agent was determined.

The results are shown in Table 2.

**Table 2**

| | Squalene absorption (mℓ/g) |
|---|---|
| Preparation Example 1 | 2.0 ± 0.1 |
| Preparation Example 2 | 3.3 ± 0.3 |
| Preparation Example 3 | 3.0 ± 0.1 |
| Preparation Example 4 | 2.5 ± 0.1 |
| Preparation Example 5 | 2.9 ± 0.1 |
| Preparation Example 6 | 0.6 ± 0.1 |
| polymer No. 14 of U.S. patent 4 489 058 | 0.6 ± 0.1 |
| talc | 0.5 ± 0.1 |
| kaolin | 0.5 ± 0.1 |

### Example 1

Scalp treatments (to be used in a shampoo-free condition) A and B of the present invention and comparative scalp treatments C, D and E having the compositions shown in Table 3 were prepared.

**Table 3**

| Formulation | Present invention | | Comparative | | |
|---|---|---|---|---|---|
| | A | B | C | D | E |
| porous vinyl polymer prepared in Preparation Example 4 | 0.3 wt% | 0.3 | 0.3 | - | - |
| Carbopol 941^{*1} | 0.18 | 0.18 | 0.18 | 0.18 | 0.18 |
| Silicone KF352A^{*2} | 0.1 | 0.1 | 0.1 | 0.1 | 0.1 |
| triethanolamine | proper amt. | proper amt. | proper amt. | proper amt. | proper amt. |
| 2-amino-2-methyl-1-propanol | proper amt. | proper amt. | proper amt. | proper amt. | proper amt. |
| Octopirox^{*3} | 0.1 | - | - | - | - |
| zinc pyrithione (Zpt) | - | 0.1 | - | 0.1 | - |
| ethanol | 50 | 50 | 50 | 50 | 50 |
| flavor | proper amt. | proper amt. | proper amt. | proper amt. | proper amt. |
| purified water | balance | balance | balance | balance | balance |
| pH control | 6 ∼ 7 | 6 ∼ 7 | 6 ∼ 7 | 6 ∼ 7 | 6 ∼ 7 |

| | | | | | |
|---|---|---|---|---|---|
| Notes) *1: carboxyvinyl polymer (a product of Goodrich) | | | | | |
| *2: polyether-modified silicone | | | | | |
| *3: Piroctone Auramine (a product of Hoechst) | | | | | |

The scalp treatments A and B of the present invention and the comparative scalp treatments C, D and E were subjected to a clinical test to examine the effects of them in controlling the formation of dandruff and greasiness of the hair.

In the clinical test, the scalp lotion D (control) was used in the first and second weeks, the scalp lotion C was used in the third and fourth weeks and the scalp lotion A (for five subjects in group 1) or scalp lotion B (for five subjects in group 2) was used in the fifth and sixth weeks. In practice, they shampooed with the control shampoo three times a week (on Monday, Wednesday and Friday). In each case, after shampooing with a control shampoo followed by drying with a towel, about 4 g of the scalp lotion was spread over the scalp. On Monday after two weeks, the formation of the dandruff and the development of greasiness of the hair were judged by the panellists and classified into four ranks. The results are shown in Table 4.

**Table 4**

| Scalp treatment | Dandruff | Greasiness |
|---|---|---|
| A | 0.8 | 0.2 |
| B | 0.2 | 0.2 |
| C | 2.8 | 1.0 |
| D | 0.2 | 3.0 |
| E | 3.0 | 2.2 |

### Example 2

Hair conditioner A of the present invention and control B having the compositions shown in Table 5 were prepared.

**Table 5**

| Formulation | A | B |
|---|---|---|
| porous vinyl polymer prepared in Preparation Example 4 | 4.5 wt% | - |
| Octopirox | 0.3 | 0.3 |
| dialkyldimethylammonium chloride | 0.5 | 0.5 |
| stearyltrimethylammonium chloride | 1.0 | 1.0 |
| solid paraffin | 0.5 | 0.5 |
| liquid paraffin | 0.5 | 0.5 |
| polymethylsiloxane | 0.5 | 0.5 |
| methylcellulose | 0.5 | 0.5 |
| propylene glycol | 3.0 | 3.0 |
| stearyl alcohol | 3.0 | 3.0 |
| colorant | proper amt. | proper amt. |
| flavor | proper amt. | proper amt. |
| purified water | balance | balance |

### Example 3

Hair preparations A, B and C of the present invention having the compositions shown in Table 6 were prepared.

**Table 6**

| | Present invention | | |
|---|---|---|---|
| | A | B | C |
| porous vinyl polymer prepared in Preparation Example 1 | 0.3 wt% | - | - |
| porous vinyl polymer prepared in Preparation Example 2 | - | 0.3 | - |
| porous vinyl polymer prepared in Preparation Example 3 | - | - | 0.3 |
| Carbopol 941 | 0.04 | 0.04 | 0.04 |
| ℓ-menthol | 0.05 | 0.05 | 0.05 |
| 2,2'-dithiobispyridine 1,1'-dioxide | 0.1 | 0.1 | 0.1 |
| flavor | 0.2 | 0.2 | 0.2 |
| ethanol | 48 | 48 | 48 |
| water | balance | balance | balance |

In Examples 2 and 3, the preparations of the present invention containing both the anti-dandruff agent and the oil-absorptive substance exhibited both the anti-dandruff and grasiness reducing effects.

### Example 4

A pasty hair preparation having the following composition was prepared:

| | |
|---|---|
| porous vinyl polymer prepared in Preparation Example 1 | 15 wt.% |
| carboxymethylcellulose | 0.5 |
| ethyl alcohol | 30 |
| flavor | 0.1 |
| water | q.s. ad 100 wt.% |

For comparison, pasty hair preparations were prepared in the same manner as above except that the polymer prepared in Preparation Example 1 was replaced with Polymer No. 14 described in the specification of U.S. Patent No. 4,489,058, talc or kaolin.

Each preparation was applied to the cheek of a subject and peeled off after one hour. The amount of sebum thus absorbed was determined by gravimetry.

The results are shown in Table 10.

**Table 10**

| | Preparation of the present invention | Polymer No. 14 of U.S. Patent No. 4,489,058 | Preparation comprising talc | Preparation comprising kaolin |
|---|---|---|---|---|
| Amount of sebum (µg/cm²) | 18 ± 6 | 2 ± 1 | 12 ± 8 | 8 ± 6 |

It is apparent from Table 10 that the preparation of the present invention has a higher capacity of absorbing sebum than the comparative ones.

## Claims

1. A hair preparation in solid form or in the form of a lotion which comprises 0.01 to 10 percent by weight of an anti-dandruff agent, 0.05 to 5 percent by weight of a cross-linked oil absorptive porous vinyl polymer in the form of particles having an average particle size of 0.005 to 30 µm and having a squalene absorption of at least 1 ml/g in the dried state and the balance of a carrier.

2. A hair preparation according to claim 1 in which said solid absorptive porous vinyl polymer is a polymer of a vinyl monomer having a solubility parameter of 7 to 10.

## Patentansprüche

1. Haarpräparat in fester Form oder in Form einer Lotion umfassend 0,01 bis 10 Gew.-% eines Antischuppenmittels, 0,05 bis 5 Gew.-% eines vernetzten ölabsorbierenden porösen Vinylpolymers in Form von Teilchen mit einer durchschnittlichen Teilchengröße von 0,005 bis 30 µm und einer Squalenabsorption von wenigstens 1 ml/g im getrockneten Zustand und einem Träger als Rest.

2. Haarpräparat nach Anspruch 1, worin das feste absorbierende poröse Vinylpolymer ein Polymer aus einem Vinylmonomer mit einem Löslichkeitsparameter von 7 bis 10 ist.

## Revendications

1. Préparation pour cheveux sous forme solide ou sous la forme d'une lotion, qui comprend 0,01 à 10 pour cent en poids d'un agent antipelliculaire, 0,05 à 5 pour cent en poids d'un polymère vinylique réticulé poreux absorbant l'huile sous la forme de fines particules ayant une taille particulaire moyenne de 0,005 à 30 µm et une capacité d'absorption du squalène d'au moins 1 ml/g à l'état séché, et le reste d'un support.

2. Préparation pour cheveux telle que revendiquée dans la revendication 1, dans laquelle ledit polymère vinylique poreux solide est un polymère dérivé d'un monomère vinylique ayant un paramètre de solubilité de 7 à 10.
